# EUROPEAN PATENT SPECIFICATION

(11) **EP 0 637 234 B1**
(45) Date of publication and mention of the grant of the patent: **02.04.1997**
(21) Application number: 93910633.2
(22) Date of filing: 19.04.1993
(51) Int. Cl.: A61F 13/56

(54) **SANITARY NAPKIN**
DAMENBINDE
SERVIETTE HYGIENIQUE

(30) Priority: 20.04.1992 JP 99131/92
(43) Date of publication of application: 08.02.1995
(73) Proprietor: MINNESOTA MINING AND MANUFACTURING COMPANY, St. Paul, Minnesota 55133-3427 (US)
(72) Inventor: IWATA, Yoji, Saint Paul, MN 55133-3427 (US)
(74) Representative: VOSSIUS & PARTNER
(86) International application number: US9303607
(87) International publication number: WO9320790

(56) References cited:
- WO-A-89/02728
- WO-A-91/16873
- US-A- 4 759 754

## Description

### Field of Invention

The present invention relates to a sanitary napkin having side panels.

### Background

Sanitary napkins comprising a liquid-permeable top sheet and a typically liquid-impermeable back sheet surrounding an absorbing member are well-known. In some instances, such napkins have first and second side panels (sometimes referred to as wings) on opposing sides. The side panels prevent fluids from flowing out of the sanitary napkin when the fluids flow from the absorbing member sideways and play a role in preventing the undergarment from being stained. In use the side panels are folded around the crotch portion of the undergarment to help secure the napkin thereto. Typically, adhesive segments are provided on the bottom sides of the side panels and on the back sheet to secure the napkin to the undergarment. WO-A-91/16873 discloses sanitary napkins with flap bonding means. US-A-4,759,754 discloses a permanent bonding of one side panel.

Japanese Unexamined Patent Publication (Kokai) No. 60-199446 discloses a sanitary napkin as shown in Figures 4 and 5 herein. In this sanitary napkin, both side panels 4, 5 have an adhesive segment 6, 7 thereon and the back sheet has adhesive segment 11 thereon. Each adhesive segment is covered by a release liner 14, 15, 13. After peeling off release liner 13 from the back sheet and securing the napkin to the crotch portion of the undergarment, the user peels off release liners 14, 15 and secures the side panels to the undergarment.

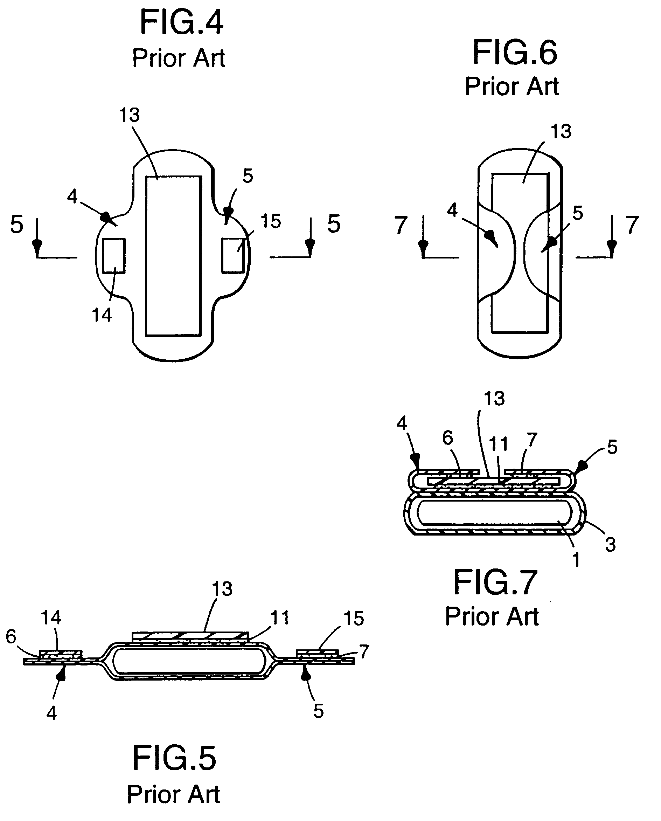

During shipping, in some instances, separate release liners are applied to each adhesive segment as shown in Figures 4 and 5. In some other instances, a single release member is used and the side panels are folded over the back sheet such that the single release member covers the adhesive segments on both side panels and on the back sheet.

Japanese Unexamined Patent Publication (Kokai) No. 63-186645 discloses a sanitary napkin wherein a single release liner covers the various adhesive segments. As shown in Figures 6 and 7 herein, release liner 13 is used to cover adhesive segments 6 and 7 on side panels 4 and 5 as well as adhesive segment 11 on the back sheet. Although this format reduces the number of release liner segments which are to be removed and disposed, the adhesive segments on the side panels are left exposed as the napkin is first applied to the undergarment and are subject to adherence to undesired locations, making application somewhat awkward.

### Summary of Invention

The present invention provides a sanitary napkin that is packaged and constructed so as to more conveniently opened, handled, and applied to an undergarment.

In brief summary, sanitary napkins of the invention comprise a liquid-permeable top sheet and a back sheet, preferably liquid-impermeable, surrounding an absorbing member and have first and second side panels on opposing sides. The first side panel has a first adhesive segment on its bottom side and the second side panel has a second adhesive segment on its bottom side. In important distinctions over previously known sanitary napkins, the side panels of sanitary napkins of the invention are folded over the top sheet such that the first and second adhesive segments are arranged side by side with a panel securing strip bonded to the first and second adhesive segments, and the side panels are secured during packaging and during use with a strip as described below. As used herein, bottom is used to refer to the side of the napkin which is oriented toward the undergarment when worn and top refers to the opposing side.

### Brief Description of Drawing

The invention will be further explained with reference to the drawing, wherein:
Figure 1 is a cross-sectional view of a sanitary napkin of the invention shown in unopened and folded position;
Figure 2 is a cross-sectional view of one embodiment of a panel securing strip used in sanitary napkins of the invention;
Figure 3A is a plane view of the sanitary napkin of Figure 1;
Figure 3B is a plane view of the sanitary napkin of Figure 3A shown partially opened and unfolded position;
Figure 3C is a plane view of the sanitary napkin of Figure 3B shown in applied position;
Figure 4 is a plane view of a sanitary napkin of the prior art shown in unopened position;
Figure 5 is a cross-sectional view of the sanitary napkin of Figure 4;
Figure 6 is a plane view of another sanitary napkin of the prior art shown in unopened and folded position; and
Figure 7 is a cross-sectional view of the sanitary napkin of Figure 6.

These figures, which are idealized, are not to scale and are intended to be merely illustrative and non-limiting.

### Detailed Description of Illustrative Embodiments

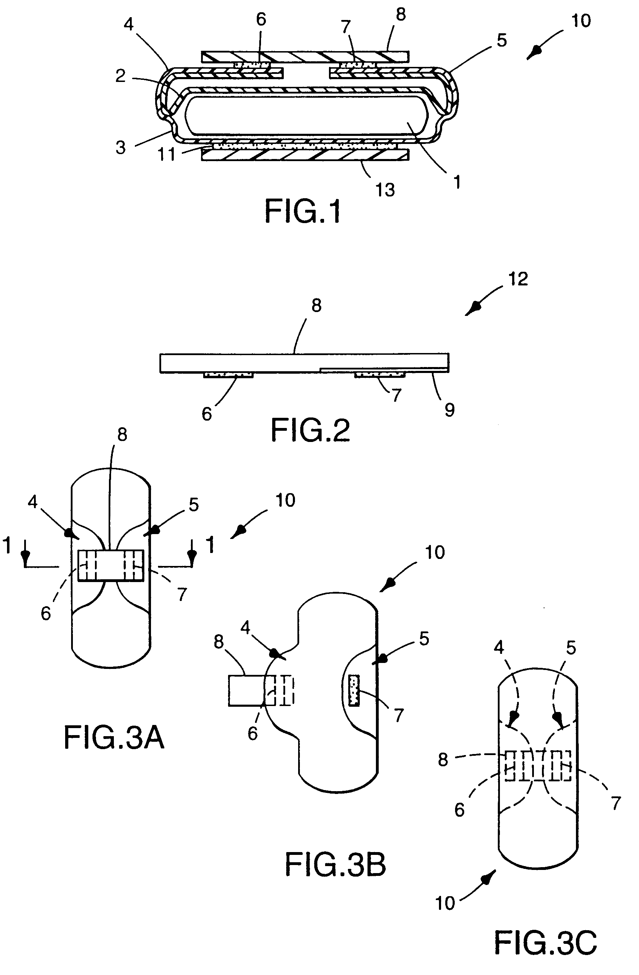

A sanitary napkin of the invention is shown in Fig. 1 wherein napkin 10 comprises liquid-permeable top sheet 2 on its front face and preferably liquid-impermeable back sheet 3 on its bottom face. Top sheet 2 and back sheet 3 surround absorbing member 1. Napkin 10 has first side panel 4 and second side panel 5 on opposing sides. First side panel 4 has first adhesive segment 6 on its bottom side and second side panel 5 has second adhesive segment 7 on its bottom side. The side panels are respectively folded over top sheet 2 such that first adhesive segment 6 and second adhesive segment 7 are arranged side by side. Napkin 10 further comprises panel securing strip 8 bonded to first adhesive segment 6 and second adhesive segment 7. Panel securing strip 8 is releasably bonded to one panel.

The materials for the top sheet, back sheet, and absorbing member can be readily selected by those skilled in the art. For instance, illustrative examples of materials useful in the absorbing member include water-absorbable polymer, pulp, absorbing paper, etc. Illustrative examples of materials useful as the liquid permeable top sheet include perforated polyolefin sheets or the like. Illustrative examples of materials useful as the liquid impermeable back sheet include polyolefin sheets, a nonwoven fabric in which a polyolefin sheet and a hydrophobic film are laminated, etc. Adherence of the top sheet to the back sheet can be carried out by any conventional means, e.g., by adhesive, heat sealing, or lamination, sewing, etc.

The adhesives used in adhesive segments 6, 7, and 11 may be any adhesive suitable for use in a sanitary napkin. Illustrative examples include synthetic and natural rubber resin adhesives. Adhesive segments 6 and 7 are preferably positioned at or near the ends of panels 4 and 5, respectively. Adhesive segment 11 is typically covered by release liner 13 during shipping and while packaged. Adhesive segments 6, 7, and 11 may be made of the same adhesive or selected separately to optimize particular performance criteria if desired.

The side panels can be conveniently made by extending the top sheet and/or back sheet. Alternatively, the side panels may be composed of a member different from either the top sheet or back sheet and be attached to the body of the napkin by conventional means, e.g., by adhesive, heat sealing, or sewing. The side panels bend at their root portions (i.e., the portions nearest the body of the napkin). When packaged, the side panels are bent over the top sheet. On those portions that face outside when so folded (i.e., the bottom surface of the side panels), adhesive segments are provided. The side panels are preferably folded so as to be symmetrically arranged. The side panel securing strip 8 is laminated over the side panels such that it covers and is bonded to adhesive segments 6 and 7.

A preferred embodiment of panel securing strip 8 is shown in Fig. 2. A portion of strip 8 is coated with release material 9 such that strip 8 will release from adhesive segment 7 when the napkin is opened and applied to an undergarment and then when the napkin is removed after use. Preferably adhesive segment 6 bonds essentially permanently to strip 8 to facilitate application of the napkin and make handling more secure. As will be understood, release material 9 may be applied, e.g., via coating, to strip 8 or extruded onto the material as strip 8 is formed. Suitable release materials may be readily selected by those skilled in the art. Strip 8 may be made from any suitable material.

During fabrication of napkins of the invention, strips 8 may be formed, preformed, typically in the form of a tape which is cut longitudinally to yield an individual tape. During assembly of the napkin it is often convenient to apply adhesive segments 6 and 7 to strip 8 and then laminate strip 8 to a napkin. If the strip with adhesive segments thereon is to be handled in roll form prior to cutting to individual strips, the surface of strip 8 opposite release material 9 may also be release coated, and preferably exhibits lesser adhesion to each of adhesive segments 6 and 7 than do the surfaces of the respective panels to which those segments are intended to be bonded.

Typically, in order to apply a napkin of the invention, release liner 13 is removed and the napkin secured to the appropriate location of the undergarment with adhesive segment 11. Panel securing strip 8 is then released from adhesive segment 7 and side panel 4 to which it is till attached is folded toward the back sheet around the crotch portion of the undergarment. Side panel 5 is folded toward the back sheet around the other edge of the crotch portion of the undergarment and adhesive segment 7 is again secured to strip 8, thus securing the napkin to the undergarment. Fig. 3A-3C illustrate the sequence of applying a sanitary napkin of the invention.

## Claims

1. A sanitary napkin (10) having a front face and a bottom face and comprising a liquid-permeable top sheet (2) on its front face and a liquid-impermeable back sheet (3) on its bottom face, said top sheet and said back sheet surrounding an absorbing member (1), said napkin having first and second side panels (4,5) on opposing sides, said first and second side panels each having a bottom side, said first side panel (4) having a first adhesive segment (6) on its bottom side and said second side panel (5) having a second adhesive segment (7) on its bottom side, said side panels being respectively folded over said top sheet such that said first and second adhesive segments are arranged side by side, characterized in that said napkin further comprises a panel securing strip (8) releasably bonded to said first adhesive segment and permanently bonded to said second adhesive segment.

2. The sanitary napkin of claim 1 further comprising one or more adhesive segments (11) on said back sheet (3).

3. The sanitary napkin of claim 2 wherein said adhesive segment (11) on said back sheet (3) is covered by a release liner (13).

## Patentansprüche

1. Damenbinde (10) mit einer Vorderseite und einer Unterseite, die an ihrer Vorderseite eine flüssigkeitsdurchlässige Decklage (2) und an ihrer Unterseite eine flüssigkeitsundurchlässige rückseitige Lage (3) aufweist, wobei die Decklage und die rückseitige Lage ein Absorptionselement (1) umgeben, wobei die Binde an gegenüberliegenden Seiten erste und zweite Seitenstreifen (4, 5) aufweist, wobei der erste und der zweite Seitenstreifen jeweils eine Unterseite aufweisen, wobei der erste Seitenstreifen (4) an seiner Unterseite ein erstes Haftsegment (6) und der zweite Seitenstreifen (5) an seiner Unterseite ein zweites Haftsegment (7) aufweisen, wobei die Seitenstreifen jeweils so über die Decklage gefaltet werden, daß die ersten und zweiten Haftsegmente nebeneinander angeordnet sind, dadurch gekennzeichnet, daß die Binde ferner ein Streifenbefestigungsband (8) aufweist, das mit dem ersten Haftsegment lösbar und mit dem zweiten Haftsegment permanent verbunden ist.

2. Damenbinde nach Anspruch 1, die ferner ein oder mehrere Haftsegmente (11) auf der rückseitigen Lage (3) aufweist.

3. Damenbinde nach Anspruch 2, wobei das Haftsegment (11) auf der rückseitigen Lage (3) durch eine Trennfolie (13) abgedeckt wird.

## Revendications

1. Serviette hygiénique (10) ayant une face frontale et une face de dessous comprenant une feuille de dessus (2) perméable aux liquides sur sa face frontale et une feuille de fond (3) imperméable aux liquides sur sa face de dessous, ladite feuille de dessus et ladite feuille de fond entourant un élément absorbant (1), ladite serviette ayant des premier et second panneaux de côté (4,5) sur les côtés opposés, lesdits premier et second panneaux de côté ayant chacun un côté de dessous, ledit premier panneau de côté (4) ayant un premier segment adhésif (6) sur son côté de dessous et ledit second panneau de côté (5) ayant un second segment adhésif (7) sur son côté de dessous, lesdits panneaux de côté étant respectivement pliés sur ladite feuille de dessus de sorte que lesdits premier et second segments adhésifs sont disposés côte à côte, caractérisée en ce que ladite serviette comprend de plus une bande (8) de fixation de panneau liée de façon libérable audit premier segment adhésif et liée de façon permanente audit second segment adhésif.

2. Serviette hygiénique selon la revendication 1, comprenant en plus un ou plusieurs segments adhésifs (11) sur ladite feuille de fond (3).

3. Serviette hygiénique selon la revendication 2, dans laquelle ledit segment adhésif (11) sur ladite feuille de fond (3) est recouvert d'un revêtement de libération (13).
